# EUROPÄISCHE PATENTSCHRIFT

(11) **EP 2 279 038 B1**
(45) Veröffentlichungstag und Bekanntmachung des Hinweises auf die Patenterteilung: **03.09.2014**
(21) Anmeldenummer: 09753909.2
(22) Anmeldetag: 27.05.2009
(51) Int. Cl.: B01D 61/28, B01D 69/10, B01D 71/12, B01D 71/82, B01D 63/08, A61M 1/16

(54) **MIKRODIALYSEMEMBRAN UND KAMMERSYSTEM MIT EINGESCHLOSSENEN KOLLOIDALEN STOFFEN UND VERFAHREN ZU DESSEN HERSTELLUNG**
MICRODIALYSIS MEMBRANE AND CHAMBER SYSTEM WITH INCLUDED COLLOIDAL MATERIALS AND METHOD FOR THE MANUFACTURE THEREOF
MEMBRANE A MICRODIALYSE ET SYSTÈME DE CHAMBRE À MATIÈRES COLLOÏDALES INCLUSES ET PROCÉDÉ POUR SA PRODUCTION

(30) Priorität: 28.05.2008 DE 102008002050
(43) Veröffentlichungstag der Anmeldung: 02.02.2011
(73) Patentinhaber: BST BIO SENSOR TECHNOLOGIE GmbH, 13156 Berlin (DE); Ehwald, Rudolf, 10115 Berlin (DE); Ehwald, Max, 10405 Berlin (DE)
(72) Erfinder: EHWALD, Rudolf, 10115 Berlin (DE); EHWALD, Max, 10405 Berlin (DE); SCHOENFUSS, Dirk, 12524 Berlin (DE); SZEPONIK, Jan, 13187 Berlin (DE)
(74) Vertreter: Gulde & Partner
(86) Internationale Anmeldenummer: PCT/EP2009/056476
(87) Internationale Veröffentlichungsnummer: WO 2009/144254

(56) Entgegenhaltungen:
- WO-A1-98/17377
- WO-A1-2006/015495
- WO-A2-03/008078
- DD-A1- 270 012
- US-A1- 2004 071 894

## Beschreibung

Die Erfindung betrifft ein Mikrodialysekammersystem, welches einen Grundkörper aus einem in Wasser nicht quellfähigen festen Stoff aufweist, mindestens einen als Dialyseraum fungierenden Hohlraum, enthaltend wasserlösliche kolloidale Stoffe sowie mindestens eine asymmetrische Kompositmembran als Dialysemembran, die den Dialyseraum abdeckt und den Grundkörper ein- oder beidseitig bedeckt. Ggf. befindet sich zwischen Grundkörper und Kompositmembran ein- oder beidseitig eine perforierte Kunststofffolie. Die asymmetrische Kompositmembran wird aus einer in Wasser nicht quellenden gerüstbildende porösen Schicht gebildet. Sie besitzt durchgehende, unverzweigte, für die kolloidalen Stoffe durchlässige Poren und ein die Poren einseitig ausfüllendes in Wasser quellfähiges organisches Xerogel, das ein Polyelektrolytkomplex aus Zellulosesulfat und Dimethyldiallylammoniumchlorid oder Chitosan ist und im gequollenen Zustand als kolloidale Stoffe Proteine mit Molmassen über 25 kDa einschließt. Die Poren der Schicht enthalten das Xerogel in ihrem Mündungsbereich auf der dem Dialyseraum abgekehrten Seite. Gegenstand der Erfindung ist auch eine für Mikrodialysekammem geeignete Dialysemembran und ein Verfahren zur Herstellung des Mikrodialysekammersystems sowie seine Verwendung.

In Mikrodialyskammern können kolloidale Sensormoleküle (z.B. Proteine, Enzyme oder andere kolloidale Rezeptoren mit Affinitätsbindungsorten) in einer diffusiblen Form eingeschlossen werden. Mikrodialysekammern, die von einer semipermeablen, für Proteine undurchlässigen und niedermolekulare Analyte permeablen Membran nach außen abgeschlossen werden, sind für die Biosensorik auf Mikrostrukturen von Interesse, weil sie die räumliche Fixierung der Sensormoleküle auf ein kleines Volumen bzw. eine kleine definierte Oberfläche in aktiver und austauschfähiger Form ermöglichen. Probleme, die mit der kovalenten Bindung der kolloidalen Sensormoleküle verbunden sind (Aktivitätsverlust, Begrenzung der flächenbezogenen Bindungskapazität, Begrenzungen in Bezug auf die Signalbildung), können dabei vermieden werden. Dennoch wird dieses Prinzip der Immobilisierung selten eingesetzt, weil es an praktikablen für die industrielle Fertigung geeigneten Techniken zur Integration von Mikrodialysekammern definierter Größe mit eingeschlossenen kolloidalen Sensormolekülen in mikrofluidisch, mikromechanisch oder optisch operierende Mikrostrukturen aus Silizium oder anderen geeigneten Feststoffen fehlt. Mikrodialysekammern haben für die Messung der Glucosekonzentration in der interstitiellen Flüssigkeit bereits Anwendung gefunden. Durchströmte Mikrodialysekammern nach dem Prinzip der Mikrodialysesonde mit nachgeschalteter Analytik bewährten sich in einem viskosimetrischen Sensor für Glucose (US 6,477, 891 B2, Ballerstädt R, Ehwald R: Biosensors & Bioeletronics 9, 557-567). Bereits vor längerer Zeit wurde vorgeschlagen, ein Sensorsystem in die Mikrodialysekammer zu integrieren, z.B. beim kompetitiven optoelektronischen Affinitätssensor für Glucose (US 4,344,438 A). In der Praxis wurden sensorisch genutzte Mikrodialysekammern für diesen Typ des optischen Affinitätssensors aus kommerziell verfügbaren Dialyse-Hohlfasern mit hydrophiler organischer Membran z.B. aus regenerierte Zellulose oder Polyethersulfon gefertigt (US 5, 143, 066 A). Die verwendeten Kapillarmembransegmente ermöglichen eine schnelle Einstellung des Diffusionsgleichgewichtes, lassen sich aber nicht problemlos im Sinne einer industriellen Fertigung in Mikrostrukturen aus Silizium, Glas oder anderen festen Stoffen integrieren, wie es beispielsweise für die industrielle Realisierung mikromechanischer Konzepte zum viskosimetrischen Glucosesensor (US 6,210,326 B1, DE 10 027 684 A1) erforderlich wäre. Das gleiche gilt für die Verwendung handelsüblicher Dialyse-Flächenmembranen. Eine hohe Permeabilität für kleine Moleküle bei gleichzeitiger Undurchlässigkeit für Kolloide wird durch Verwendung von Membranen erreicht, die durch eine hohe Quellfähigkeit in Wasser ausgezeichnet sind. Sie bestehen aus gelartigen hydrophilen organischen Substanzen, deren Integration in mikromechanische, optische bzw. mikrofluidische Mikrostrukturen wegen starker quellungsbedingter Volumen- oder Flächenänderungen problematisch ist. Mikrofiltrationsmembranen mit Porenweiten über 20, meist über 100 nm, die aus nicht quellfähigen Stoffen bestehen, sind in zahlreichen Ausführungsformen bekannt. Diese Membranen besitzen aber nicht die für die Herstellung einer breit einsetzbaren Mikrodialysekammer erwünschten Merkmale einer hohen Permeabilität für die Diffusion von niedermolekularen Analyten und einer definierten Größenausschlussgrenze.
Kompositmembranen, bei denen das porenfüllende Gel in der Membran mehr oder weniger gleichmäßig verteilt ist, (Mika A. M., Childs R. F., Dickson J.M., McCarry B.E. und Gagnon D.R., Journal of Membrane Science 108, 37-56, 1995, U.S. Patent No. 6.258.276) sind als Dialysemembranen ungeeignet. Es ist bereits seit 1877 durch Wilhelm Pfeffer bekannt, dass semipermeable asymmetrische Kompositmembranen durch Bildung einer dünnen kontinuierlichen Schicht aus einer gelförmigen anorganischen Substanz (kolloidales Berliner Blau) in den vernetzten Hohlräumen einer rigiden Mikrofiltermembran (poröse Keramik) gebildet werden können. Bei der semipermeablen Membran der Pfefferschen Zelle handelt es sich um eine asymmetrische Kompositmembran, bei der die mikroporöse Stützschicht aus einem rigiden, nicht quellfähigen Material und die Trennschicht aus einer kolloidalen bzw. gelartigen, in Wasser quellfähigen anorganischen Substanz besteht. Asymmetrische Kompositmembranen mit mikroporöser, rigider Stützschicht sind in verschiedenen Ausführungsformen bekannt. Sie können eine einseitig aufgelagerte gelartige Deckschicht besitzen (z.B. Decher und Hong, Bunsenges. Phys. Chem. 95, 1430,1991). Hiervon zu unterscheiden sind asymmetrische Kompositmembranen, bei denen die gelartige Substanz in die Poren der Stützschicht integriert ist wie in der Membran der Pfefferschen Zelle. Dieser Aufbau der Membran hat den Vorteil, dass die Dicke der Membran von der Quellung des Gels nicht abhängt und die Quellung des Gels in Wasser durch die rigide Porenwand begrenzt wird. Hierdurch kann die Gelkonzentration und damit die Ausschlussgrenze genau definiert werden.

EP 1409 119 B1 beschreibt den Aufbau einer asymmetrischen Kompositmembran, die aus einer mikroporösen rigiden Stützschicht bzw. Mikrofiltermembran mit vernetzten Poren und einer im vernetzten Porensystem verankerten kontinuierlichen oder im Wesentlichen kontinuierlichen Trennschicht besteht. Die Trennschicht wird aus einem kovalent vernetzten organischen, quellfähigen Gel mit asymmetrischer bzw. einseitiger Dichteverteilung in der Membran gebildet. Der in EP 1409 119 B1 dargestellte Membranaufbau umfasst auch solche Membranen, bei denen die kontinuierliche Gelschicht dünner ist als die poröse Stützschicht, so dass auf einer der Membran - Hauptflächen die Mikroporen gelfrei sind. Die in EP 1409 119 B1 beschriebenen Membranen wurden für die Ionenfiltration entwickelt und besitzen einen schwerwiegenden Nachteil für den Einsatz zur Dialyse an Mikrostrukturen. Die quervernetzten Poren der Mikrofiltermembran, welche die Bildung einer kontinuierlichen Gelschicht durch kovalente Vernetzung von hydrophilen Polymeren oder dreidimensionale Polymersiation ermöglichen, sind in dem durch das Gel nicht ausgefüllten Teil der Stützschicht für die laterale Diffusion der einzuschließenden Stoffe wegsam. Bei Verwendung als Dialysemembran bewirkt die laterale Diffusion der in der Mikrodialysekammer eingeschlossenen Makromoleküle ihre allmähliche Verdünnung in der Mikrodialysekammer bzw. die unerwünschte allmähliche Ausbreitung ihrer Wirkungsfläche auf der Mikrostruktur. Membranen, die sich für die Dialyse an Mikrostrukturen eignen, stehen bisher nicht zur Verfügung.

WO 2006/015495 A2 offenbart Kompositmembranen aus einem nicht quellbaren PP-Support mit durchgehenden Poren, die asymmetrisch mit einem quellbaren Gel beladen sind. US 2004/071894 A1 beschreibt die Verwendung eines porösen Trägers mit einem Xerogel zur Herstellung asymmetrischer Membranen. Aus DD 270 012 A1 sind poröse Träger mit Polyelektrolytmembranen bekannt. In WO 98/17377 A1 sind Membranen für Trennverfahren umfassend ein poröses Substrat und ein in den Poren vernetztes Hydrogel oder ein Polyelektolyt beschrieben.

Die Aufgabe der Erfindung besteht darin, eine Mikrodialysekammer bereitzustellen, deren Membran hochpermeabel für niedermolekulare Analyte ist und die andererseits kolloidale Stoffe, wie z.B. Sensormoleküle zuverlässig und sicher in der Mikrodialysekammer zurückhält. Dabei soll die Mikrodialysekammer ein definiertes Verteilungsvolumen oder eine definierte Wirkungsfläche für die eingeschlossenen Kolloide besitzen und sich leicht in z.B. vorgefertigte Mikrostrukturen aus z.B. Silizium, Glas oder Kunststoff und dergleichen integrieren lassen. Die Aufgabe der Erfindung bestand weiter darin, universell einsetzbare Mikrodialysemembranen bereitzustellen, die durch eine hohe Permeabilität für niedermolekulare Stoffe und eine scharfe Größenausschlussgrenze gekennzeichnet sind. Sie sollen insbesondere in der Lage sein, Makromoleküle im Größenbereich der Proteine (Stokes' Radius größer als 2 nm) langfristig von der Diffusion auszuschließen. Sie sollen auch bei langen Diffusionszeiten einen perfekten Einschluss von z.B. Proteinen mit Molmassen über 25 kDa bzw. von Makromolekülen mit einem Stokesschen Radius über 2 nm gewährleisten.

Die Aufgabe wird durch ein Mikrodialyse membrane nach Anspruch 1 und einem Verfahren zur Herstellung eines Mikrodialyse Kammersystems nach Anspruch 10 gelöst. Die Unteransprüche betreffen vorteilhafte Ausführungsformen der Erfindung.

Die Erfindung betrifft eine Membran für ein Mikrodialysekammersystem, welches einen festen Grundkörper 1 aus einem in Wasser nicht quellfähigen festen Stoff, ggf. eine perforierte Kunststofffolie 2, mindestens einen so genannten Dialyseraum 4 und eine asymmetrische Kompositmembran 3 umfasst. Die Kompositmembran 3 besteht aus einer gerüstbildenden porösen Schicht 7 aus in Wasser nicht quellfähigem rigidem Material mit unverzweigten und unvernetzten Poren und einem in Wasser quellfähigen organischen Xerogel 6 aus Zellulosesulfat und Dimethyldiallylammoniumchlorid oder Chitosan, das sich einseitig (nicht durchgängig) in den Poren der Schicht 7 befindet und im gequollenen Zustand eine Permeationsbarriere für kolloidale Stoffe 5, welche Proteine mit Molmassen über 25 kDa sind, darstellt.

Das Merkmal in Wasser nicht quellfähig wird in dieser Schrift auf alle festen Stoffe angewendet, die ihre Ausdehnung bei vollständiger Benetzung mit Wasser wegen ihrer rigiden Struktur nicht stärker als 1 % ändern. Die Verwendung nicht quellfähiger Stoffe als Material der porösen Schicht gewährleistet die Volumenkonstanz der Dialysekammer und die Konstanz der Austauschfläche an der Dialysemembran. Die Verwendung dünner poröser Stützschichten mit unverzweigten Poren ist mit der Ausbildung einer in der Membran verhakten kontinuierlichen oder im Wesentlichen kontinuierlichen Gelschicht an sich unvereinbar. Überraschend zeigte sich jedoch, dass eine stabile Befestigung des Gels ohne kovalente Bindung an den Feststoff in unvernetzten Poren möglich ist, wie in den Ausführungsbeispielen noch näher ausgeführt wird. Der Vorteil der Verwendung unvernetzter Poren besteht darin, dass die Diffusion der eingeschlossenen kolloidalen Stoffe nur senkrecht zu den Hauptflächen der Membran erfolgen kann.
Rigide bedeutet in dieser Schrift im Sinne einer Stoffeigenschaft, dass der verwendete Stoff eine im Vergleich mit regenerierter Zellulose wesentlich höhere Steifigkeit und Biegefestigkeit besitzt.
Die Verwendung einer asymmetrischen Kompositmembran mit nur einseitig und teilweise gefüllten Poren für die Entwicklung von flächen- und volumenkonstanten Diälysemembranen hat den großen Vorteil, dass die diffusionsbehindernde Zone in den Poren kürzer gestaltet werden kann.

Durch mindestens eine Kavität im Grundkörper 1, durch dessen Perforation, durch die perforierte Folie 2 und/oder in den freien Poren der porösen Schicht 7 wird mindestens ein Hohlraum gebildet, der mit wasserlöslichen kolloidalen Stoffen 5 gefüllt ist, und der nach Abdeckung mit der Kompositmembran 3 den Dialyseraum 4 bildet. Die Schichten sind dabei so miteinander verbunden, dass die Kompositmembran 3 den Grundkörper 1 mit dem mindestens einen Dialyseraum 4 abdeckt, wobei jede Pore der Schicht 7 das Xerogel 6 nahe ihrer Mündung auf der dem Dialyseraum 4 abgekehrten Seite enthält. In einer Ausführungsvariante befindet sich auf dem Grundkörper 1 ein- oder beidseitig eine perforierte Kunststofffolie 2. Durch die Struktur (Perforation) der Folie kann mindestens ein Dialyseraum 4 gebildet werden, welcher die kolloidalen Stoffe 5 in entwässertem Zustand einschließt. Die asymmetrische Kompositmembran 3, welche den mindestens einen Dialyseraum 4 mit den kolloidalen Stoffen 5 abdeckt, besteht aus der rigiden in Wasser nicht quellenden gerüstbildenden porösen Schicht 7, die 20 bis 120 µm dick ist und dem in Wasser quellfähigen organischen Xerogel 6, wobei die Poren durchgehend und unverzweigt sind und das Xerogel 6 sich in den Poren an nur einer Seite - im System in den Poren an der Außenseite bis zu einer Tiefe von 10 µm - befindet. Die asymmetrische Kompositmembran ist dichtend ein- oder beidseitig auf dem Grundkörper 1 ggf. auf der Folie 2 fixiert. Da die Poren durchgehend, 20 bis 1000 nm weit und nicht miteinander verbunden sind, sowie der Anteil der Porenfläche an der Hauptfläche der porösen Schicht über 25 % beträgt, bildet das in den Poren nahe der Außenfläche der Membran einseitig verankerte organische Xerogel keine kontinuierliche Schicht. Besonders bevorzugt füllt das Xerogel (6) die Poren bis zu einer Tiefe von 5 µm. aus. Die bevorzugte Weite der Poren liegt zwischen 50 bis 250 nm, ein bevorzugter Anteil von Porenfläche an der Hauptfläche der porösen Schicht beträgt mehr als 40%, besonders bevorzugt liegt er zwischen 40 und 80%.

Erfindungsgemäß ist das in den Poren im Nahbereich einer der Membranflächen vorliegende Xerogel ein Polyelektrolytkomplex aus Zellulosesulfat (Polyanion) und Dimethyldiallylammoniumchlorid oder Chitosan (Polykation). Durch die Wahl der Molekülgröße der Polykationen und Polyanionen und ihre Konzentration können mit einfachen Mitteln Polyelektrolytkomplexe definierter Dicke und definierter Dichte hergestellt werden. Die Größenausschlussgrenze dieser Polyelektrolytkomplexe kann hierdurch so eingestellt werden, dass ein für die Mikrodialyse ideales scharfes Langzeit-Cut-off mit einer Größenausschlussgrenze von etwa 2 nm (Stokesscher Radius) entsteht. Überraschend hat sich gezeigt, dass die Polyelektrolytkomplexe, die in den unverzweigten Poren einer Mikrofiltermbran in bekannter Weise aus der Reaktion des Polyanions mit dem Polykation gebildet wurden, auch ohne zusätzliche Befestigung irreversibel in der Membran verankert sind, auch wenn sie keine kontinuierliche Schicht im Sinne von EP 1 409 119 B1 bilden können. Näheres hierzu wird im Beispiel 1 ausgeführt.
Der Vorteil der Verwendung der Polyelektrolytkomplexe zur asymmetrischen Porenfüllung bei der Bildung der erfindungsgemäßen Mikrodialysemembran besteht in einer optimalen Barrierefunktion für z.B. eingeschlossene Proteine mit relativ kleinem Molekulargewicht in der nachgewiesenen hohen Permeabilität für niedermolekulare Stoffe und in einem vergleichsweise geringen Aufwand für die Bildung des trennwirksamen Gels in die Poren im Nahbereich einer der Membranhauptflächen.

Der Dialyseraum 4 des Mikrodialysekammersystems kann durch das Volumen der Poren der asymmetrischen Kompositmembran realisiert werden oder als flache Vertiefung bzw. Kavität in die Feststoffoberfläche des Grundkörpers 1 mit modernen Techniken der Mikrostrukturierung gestaltet werden, er kann durch Perforieren des Grundkörpers 1 oder mittels perforierter Zusatzfolie 2 bzw. im unteren Teil der Poren der Kompositmembran 3 erzeugt werden. Dieser Dialyseraum 4 enthält erfindungsgemäß die kolloidalen Stoffe 5 in entwässerter Form. Sie haften trocknungsbedingt bevorzugt entweder in der Kavität oder in dem an das Lumen des Dialyseraums grenzenden Areal der asymmetrischen Kompositmembran.

Ein wichtiges Merkmal der Erfindung ist die als asymmetrische Kompositmembran 3 gestaltete Dialysemembran. Erfindungswesentlich ist ihr Aufbau aus der gerüstbildenden porösen Schicht 7, deren Poren durchgehend und unverzweigt sind und die aus in Wasser nicht quellendem rigiden Material gebildet ist, und dem in Wasser quellenden hydrophilen organischen Xerogel 6 aus Zellulosesulfat und Dimethyldiallylammoniumchlorid oder Chitosan, das sich nur auf der dem Lumen des Dialyseraums 4 abgewandten Seite der Kompositmembran 3 befindet. Das Xerogel 6 bildet bei der Quellung mit Wasser eine in den Porenausgängen bzw. im Mündungsbereich jeder Pore verankerte semipermeable Trennzone. Das Xerogel hat nur einen geringen Anteil an der Masse der Kompositmembran. Vorzugsweise sind die Poren der Schicht 7 auf dem größten Teil ihrer Länge frei von diesem Gel. Das Xerogel füllt nach Einführen in eine wässrige Matrix aufgrund der erfindungsgemäßen Zusammensetzung der Kompositmembran die Poren im äußeren Mündungsbereich quellungsbedingt vollständig aus. Es bildet dort in jeder Pore eine dünne Trennzone mit scharfer Ausschlussgrenze und behindert daher die Permeation kleiner Moleküle nicht stark. Die gerüstbildende poröse Schicht 7 der Kompositmembran 3 besteht aus einem festen, in Wasser nicht quellfähigen porösen Stoff mit hohem Porenvolumenanteil unselektiver Poren, in denen der Diffusionskoeffizient für gelöste Stoffe nicht oder wenig gegenüber der Diffusion in unbewegtem Wasser verringert ist.

Die Distanz zwischen der äußeren Oberfläche der Kompositmembran und dem Grund des Dialyseraums beträgt vorzugsweise ≤ 0,6 mm, d.h. der gesamte Diffusionsweg durch das Xerogel, die gelfreie Porenzone und die ruhende Flüssigkeit im Dialyseraum ist i.d.R. kürzer als 600 µm und gewährleistet so eine relativ kurze Zeit für die Einstellung des Diffusionsgleichgewichtes eines niedermolekularen Analyten und des Wassers. Die notwendige Stärke der gerüstbildenden porösen Schicht hängt, falls Biegung durch osmotisch bedingte Druckdifferenzen vermieden werden soll, von der maximalen Weite der Dialysekammer und von dem Biegungselastizitätsmodul der porösen Schicht ab.

Die Kompositmembran 3 weist erfindungsgemäß eine Dicke zwischen 20 bis 120 µm auf, das Xerogel (6) füllt die Poren nur bis zu einer Tiefe von 10 µm aus. Weiterhin sind die Poren durchgehend und unverzweigt sowie 20 bis 1000 nm weit und der Anteil der Porenfläche an der Hauptfläche der porösen Schicht beträgt über 25 %, wobei das Xerogel (6) im gequollenen Zustand eine Permeationsbarriere für die kolloidalen Stoffe (5) darstellt, während es für niedermolekulare Stoffe permeabel ist. Da sie das quellfähige Xerogel nur in den Poren einer Seite enthält, kann die asymmetrische Kompositmembran mit der inneren Seite problemlos und dauerhaft auf nichtquellfähigen Feststoffen fixiert werden.

In Abhängigkeit vom Anwendungszweck weist der Grundkörper ggf. eine sensorische Mikrostruktur auf. Dabei kann es sich um Strukturen zum Einsatz in einem optischen, mikromechanischen oder elektrochemischen Sensorsystem handeln. Bevorzugt besteht er aus Kunststoff, Glas, Silizium oder Metall.

Die gerüstbildende poröse Schicht 7 der asymmetrischen Kompositmembran 3 besteht aus einem rigiden, mit Wasser nicht quellenden Stoff, der sich für dauerhaftes und wasserfestes Kleben oder Bonden eignet und besitzt unverzweigte durchgehende Poren. Je nach der Art des für die Mikrostruktur verwendeten Materials kann diese Schicht der asymmetrischen Kompositmembran z. B. aus keramischem Material, Metall, Metalloxid, in der Halbleiterindustrie üblichem Material wie z.B. Si, SiO₂, Metall oder nicht quellfähigem Kunststoff wie z.B. Nylon bestehen. Für die Verwendung als gerüstbildende poröse Schicht der erfindungsgemäß eingesetzten asymmetrischen Kompositmembran geeignete dünne Mikrofiltermembranen sind auf unterschiedlicher chemischer Grundlage handelsüblich. Für die Integration in ein erfindungsgemäßes Mikrodialysekammersystem sind bevorzugt Schichten aus keramischen Material, Metall, Metalloxid, Silizium, Siliziumdioxid, oder einem nicht quellfähigen Kunststoff geeignet, vorausgesetzt sie besitzen durchgehende, unverzweigte unselektive Poren und sind hochpermeabel für niedermolekulare Analyte.

Besonders bevorzugt wird als poröse Schicht eine Mikrofiltermembran aus einem festen, mit Wasser nicht quellfähigen porösen Stoff mit einem Porenvolumenanteil > 25 % und einer Porenweite ≥ 50 nm eingesetzt, vorzugsweise eine Mikrofiltermembran mit einem mittleren Porendurchmesser von 50 bis 250 nm und einer Dicke von 50 bis 100 µm. Besonders bevorzugt sind diese Membranen aus Metalloxid, z.B. aus Aluminiumoxid. Weitere bevorzugte Materialien sind Silizium oder Edelmetalle wie z. B. Silber und Gold.

So kann z.B. bevorzugt eine asymmetrische Kompositmembran verwendet werden, deren poröse Schicht eine elektrolytisch erzeugte Mikrofiltermembran ist, in welche das Xerogel eingebracht wird. Diese Mikrofiltermembranen sind handelsüblich, z.B. Mikrofiltermembranen mit einer Dicke von 60 µm und genau definierten Porenweiten von 100 oder 200 nm. Das nicht quellfähige rigide Feststoffgerüst solcher Mikrofiltermembranen besteht z.B. aus inertem kristallinem Aluminiumoxid und ist von eng beieinander liegenden unvernetzten und unverzweigten Mikroporen durchzogen. Dünne, rigide Mikrofiltermembranen mit hohem Flächanteil (> 50 %) von 50 - 250 nm weiten unverzweigten Poren, werden bevorzugt zur Herstellung von Kompositmembranen für das erfindungsgemäße Mikrodialysekammersystemeingesetzt.

Das einseitig im Mündungsbereich ihrer Poren verankerte organische Xerogel ist ein Polyelektrolytkomplex aus Zellulosesulfat und dem Polykation Poly-Dimethyldiallylammoniumchlorid oder Chitosan, der sowohl auf der dem Dialyseraum zugewandten Seite als auch auf der dem Dialyseraum abgewandten Seite mit Zellulosesulfat abgesättigt und dadurch negativ geladen ist. Ein solcher Polyelektrolytkomplex füllt im gequollenen Zustand die Poren auf einer Strecke von vorzugsweise maximal 10 µm, besonders bevorzugt von 2 - 5 µm aus, und maskiert einseitig die kristalline Membranoberfläche der so gebildeten Kompositmembran.
Die stabile einseitige Befestigung des Gels in den unvernetzten Poren wird erreicht, indem bei der Bildung des Polyelektrolytkomplexes die in mit dem Aufbau der Membran verbundene Anisotropie der Diffusion der Reaktionspartner in den durchgehenden unverzweigten Poren, die räumliche Begrenzung der Reaktion durch Polymerdiffusion und Gelbildung sowie die überraschende Haftung des Polyelektrolytkomplexes in den Poren ausgenutzt wird.

Das erfindungsgemäß gestaltete Mikrodialysekammersystem ermöglicht den Einschluss der kolloidalen Stoffe, die zur Analytik genutzt werden können.
Beispielsweise werden kolloidale Sensormoleküle eingeschlossen, welche sich zum Einsatz in einem optischen, mikromechanischen oder elektrochemischen Sensorsystem eignen. Beispielsweise sind die kolloidalen Sensormoleküle Affinitätsrezeptoren, Enzyme, Lektine, Antikörper u.a. Kolloide mit Affinitätsbindungszentren, die ohne Verlust ihrer Bindungsfähigkeit aus Wasser getrocknet werden können.
Alternativ kann das erfindungsgemäße Mikrodialysekammersystem auch zur Fertigung eines Membranosmometers genutzt werden, wobei die osmotische Wirksamkeit eingeschlossenen Kolloide ausgenutzt wird. Vorzugweise werden in diesem Fall neutrale Hydrokolloide wie Polyethylenglycol oder Dextran im Dialyseraum eingeschlossen. Sie erzeugen einen Überdruck im Dialyseraum, der nicht vom Ionengehalt der Lösung abhängt und der selektiv von der äußeren Polymerkonzentration abhängt. Das Mikrodialysesystem muss für diese Anwendung einen Drucksensor enthalten. Es kann in dieser Form z.B. für die Messung der Wasserdampfaktivität in einer Gasatmosphäre oder die Bestimmung der Molmasse von Makromolekülen eingesetzt werden.

Gegenstand der Erfindung ist auch die Herstellung des Mikrodialysekammersystems. Die Herstellung der erfindungsgemäßen Mikrodialysekammer umfasst im Prinzip die folgenden Arbeitsschritte:
- Strukturierung bzw. Bereitstellung eines Hohlraumes zur Aufnahme kolloidaler Stoffe zur Schaffung eines Dialyseraums.
- Dosieren der kolloidalen Stoffe und
- Verschluss des Dialyseraums.
In einer Vorzugsvariante erfolgt das Dosieren der kolloidalen Stoffe in gelöster bzw. dispergierter Form, wobei die flüssigen Bestandteile vor dem Verschluss des Dialyseraums verdunsten, so dass die kolloidalen Stoffe beim Verschluss des Dialyseraums in entwässerter Form vorliegen.

Das Verfahren ist im wesentlichen dadurch gekennzeichnet, dass kolloidale Stoffe 5 in mindestens einen Dialyseraum 4 oder auf die vom Xerogel 6 freie Oberfläche der Kompositmembran 3 in der zum Abdecken eines Dialyseraums 4 vorgesehenen Position in fester oder flüssiger Form dosiert werden, ggf. die Flüssigkeit verdunstet wird und die asymmetrische Kompositmembran 3 über den Grundkörper mit dem mindestens einen Dialyseraum 4 dichtend z.B. mit Hilfe eines Klebstoffes, einer Klebefolie oder durch Lasern fixiert wird.

Die Strukturierung des Grundkörpers 1 mit mindestens einem Hohlraum zur Bildung des Dialyseraums 4 kann entweder durch Herstellung einer Vertiefung auf der Oberfläche des Grundkörpers 1, durch Perforieren oder durch Integration einer perforierten Folie erfolgen. Das Dosieren der kolloidalen Stoffe erfolgt z.B. durch Auftragen eines oder mehrerer Mikrotropfen mit anschließender Trocknung oder durch Einfügen z.B. vorgefertigter Pellets mit getrockneten kolloidalen Stoffen oder z.B. durch Verwendung vorgefertigter passender Flächenträger, z.B. aus Zellulose oder Nylon, mit adherierten getrockneten kolloidalen Stoffen. Der Verschluss des Mikrodialysekammersystems erfolgt z.B. durch Kleben oder Bonden. Wird die Mikrodialysekammer durch Perforation einer Folie strukturiert, kann letztere vor dem Dosieren der kolloidalen Stoffe mit der asymmetrischen Kompositmembran an die plane Fläche einer Mikrogrundstruktur geklebt oder gebondet werden. Alternativ ist es möglich, zuerst die asymmetrische Kompositmembran mit der perforierten Folie zu verbinden. Der Abschluss des Mikrodialysekammersystems nach dem Dosierschritt wird in beiden Fällen durch Verschluss der noch offenen Seite der Kavität erreicht, wobei zu gewährleisten ist, dass der Hohlraum der Mikrodialysekammer mindestens einseitig von der asymmetrischen Kompositmembran begrenzt wird und dass die gelfreie Fläche dieser Membran an den Hohlraum grenzt.

Liegt der Grundkörper perforiert vor, wobei die Perforation den Dialyseraum bildet, der die kolloidalen Stoffe enthält, kann der Grundkörper beidseitig durch die Kompositmembran abgedeckt sein oder je nach gewünschten Einsatzgebiet auf einer Seite durch die asymmetrische Kompositmembran und auf der anderen Seite durch dichtende Materialien abgedeckt werden.

Die dichtende Fixierung der Kompositmembran 3 am Grundkörper 1 bzw. ggf. an der Folie 2 kann dem Einschluss der zuvor in die offene Kavität dosierten kolloidalen Stoffe 5 dienen. Die dichtende Fixierung der Kompositmembran 3 an eine perforierte Folie 2 kann der Bildung einer Kavität 4 dienen, in welche die kolloidalen Stoffe eingebracht und ggf. entwässert werden, bevor die perforierte Folie 2 mit der befüllten Kavität 4 an die Mikrogrundstruktur 1 fixiert wird.

Dabei kann eine Lösung sensorisch wirksamer Kolloide zusätzliche schützende hydrophile und quellfähige Kolloide, z.B. Dextran mit hoher Molmasse zur Verbesserung der Kompatibilität des Trocknungsvorganges und zur Erleichterung und Beschleunigung der Auflösung enthalten. Das Vorliegen der kolloidalen Stoffe in trockener Form erleichtert die Handhabung des Mikrodialysekammersystems, z.B. dessen Lagerung und Entkeimung, und ermöglicht ein problemloses Aufbringen der asymmetrischen Kompositmembran durch z.B. Bonden oder Kleben.

Das erfindungsgemäße Mikrodialysekammersystem mit eingeschlossenen kolloidalen Stoffen, welches bevorzugt mit einem optischen, mikromechanischen oder elektrochemischen Sensorsystem versehen ist, kann in der Analytik von niedermolekularen Stoffen verwendet werden, wenn die kolloidalen Stoffe die niedermolekularen Analyte selektiv binden. Da für die meisten komplex gebauten organischen Analyte, z.B. Zucker, organische Säuren, Aminosäuren oder Steroide, kolloidale Rezeptormoleküle bekannt sind, kann das erfindungsgemäße Mikrokammersystem in der Analytik breit eingesetzt werden. Die Langzeit-Größenausschlussgrenze der erfindungsgemäß verwendeten Kompositmembran liegt vorzugsweise bei einem Stokesschen Radius von etwa 2 nm. Hierdurch ist gewährleistet, dass Proteine durch die erfindungsgemäß verwendete asymmetrische Kompositmembran effektiv zurückgehalten werden, während niedermolekulare Analyte, wie z.B. Glucosemoleküle sehr schnell permeieren. Die erfindungsgemäße Membran kann ohne irreversible Veränderung ihrer Semipermeabilität getrocknet werden, z.B. aus Wasser oder Ethanol. Wird ein solches Mikrodialysekammersystem mit dieser Membran in eine entgaste wässrige Lösung eingebracht, lösen sich in kurzer Zeit das eingeschlossene Gas und die eingeschlossenen kolloidalen Stoffe, wie z.B. entsprechende Sensormoleküle, in der eingedrungenen Lösung auf. Erfindungsgemäß ist jede asymmetrische Kompositmembran geeignet, die aus einer dünnen, in Wasser nicht quellenden, porösen Schicht und einem organischen hydrophilen und im gequollenen Zustand semipermeablen organischen Xerogel besteht. Entscheidend ist, dass dieses Xerogel auf einer Seite der Membran im Porenraum verankert ist, während in den gelfreien Porenraum auf der anderen Seite der Membran z.B. der Klebstoff eindringen kann. Die asymmetrische Verankerung des organischen Xerogels in den Poren auf der nach außen exponierten Seite der gerüstbildenden porösen Schicht und die hierdurch bedingte Maskierung des gerüstbildenden Feststoffes bietet vielfältige Möglichkeiten für die Schaffung einer biokompatiblen Oberfläche, auch wenn der gerüstbildende Feststoff in unmaskierter Form unerwünschte Gewebsreaktionen (z.B. Bewuchs mit Fibroblasten, Beschichtung mit Proteinen) fördert.

Die geringe Tiefe der Kavität ermöglicht in Zusammenhang mit der hohen Wasserdurchlässigkeit des Xerogels und der Quellfähigkeit und Löslichkeit der eingeschlossenen entwässerten Kolloide eine spontane Füllung des Mikrodialysekammersystems, wenn das letztere mit Wasser in Verbindung gebracht wird. Die Zugabe geeigneter stark quellender Hydrokolloide wie z.B. Dextran oder Polysucrose in die einzubringende Lösung der bevorzugt verwendeten kolloidalen Sensormoleküle kann sich vorteilhaft auf die Erhaltung der biologischen Aktivität beim Trocknen und auf die Geschwindigkeit der Auflösung der entwässerten Moleküle beim Eindringendes Mikrodialysekammersystems in Wasser auswirken.

Befindet sich in der Mikrodialysekammer nach dem Einbringen in Wasser noch eingeschlossene Luft, so wird diese leicht durch Wasser verdrängt, wenn das Mikrodialysekammersystem in eine entgaste wässrige Lösung eingebracht wird.

Wesentliche Vorteile der Erfindung sind:
- praktikable Integrierbarkeit kolloidaler Stoffen in Mikrostrukturen an Feststoffoberflächen,
- effizientes und verlustfreies Einbringen oder Immobilisieren von Kolloiden im löslichen und austauschfähigen Zustand bei definierter Schichtdicke im Mikrometerbereich,
- reproduzierbares, praktikables Herstellungsverfahren,
- schnelle Auflösung und Verteilung der kolloidalen Stoffe nach dem Einbringen in Wasser.

### Ausführungsbeispiele:

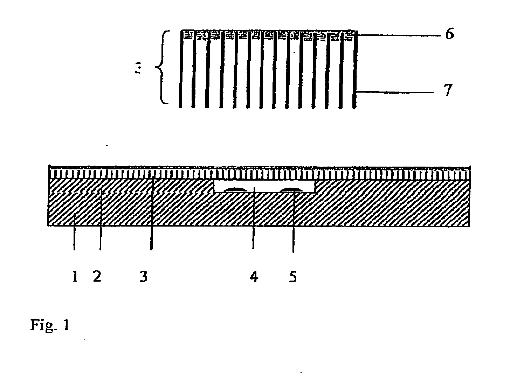

- Fig. 1 -: Grundkörper 1 mit Kavität 4 (enthaltend kolloidale Stoffe 5) und Kompositmembran 3

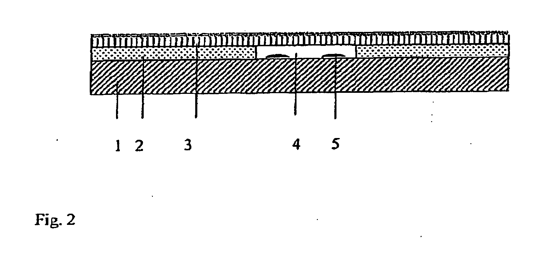

- Fig. 2 -: Grundkörper 1 mit perforierter Folie 2 (enthaltend kolloidale Stoffe 5) und Kompositmembran 3
- Fig. 3 -: Osmometer umfassend einen perforierten Grundkörper 1 (enthaltend kolloidale Stoffe 5) und Kompositmembran 3
- Fig. 4 -: zeigt die Abhängigkeit des Retentionsfaktors von der Molekülgröße(Stokesscher Radius) nach 14 tägiger Dialyse einer polydispersen Dextranmischung durch die asymmetrische Kompositmembran. Die Membran ist für Moleküle mit einem Stokesschen Radius über 1,5 nm vollkommen unpermeabel.

### Legende zu den Abbildungen

- 1: Grundkörper
- 2: Kunststofffolie
- 3: Kompositmembran
- 4: Dialyseraum
- 5: kolloidale Stoffe
- 6: organisches Xerogel
- 7: gerüstbildende poröse Schicht
- 8: elektrisch isolierte Biegeplatte
- 9: plattenförmiger Körper
- 10: elektrische Leiter
- 11: elektrische Anschlüsse

### Beispiel 1

### Herstellung eines Mikrodialysekammersystems

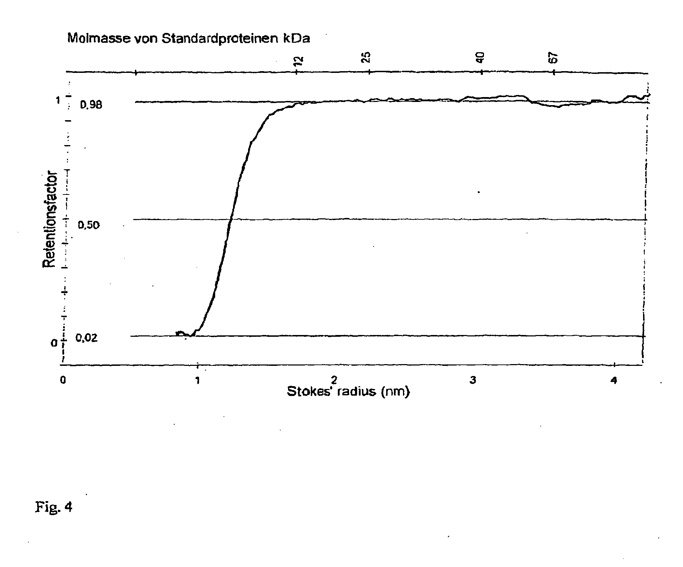

Eine poröse asymmetrische Kompositmembran, deren gerüstbildende poröse Schicht eine handelsübliche 60 µm dicke Mikrofiltermembran aus Aluminiumoxid (Anodisk^{®}, Hersteller Whatman, USA) mit einer Porenweite von 100 oder 200 nm ist, wurde durch asymmetrische Beladung der Poren dieser Schicht mit einem organischen Xerogel hergestellt. Ein außen negativ geladener Elektrolytkomplex aus Zellulosesulfat und Poly-Diallyldimethylammoniumchlorid wurde im Mündungsbereich der Poren an einer Membranseite in die Mikrofiltermembran integriert. Er bildet das Xerogel. Der gequollene Polyelektrolytkomplex füllt die Poren der Aluminiumoxidschicht auf einer Länge von etwa 3 µm, was mit Hilfe des Fluoreszenzmikroskops durch Adsorption des Fluoreszenzfarbstoffes Calcofluor White an das Zellulosesulfat an Querschnitten der Kompositmembran festgestellt wurde. Die Größenausschlussgrenze der asymmetrischen Kompositmembran entspricht einem Stokesschen Radius von etwa 2 nm, was mit Hilfe eines chromatographischen Verfahrens (Woehlecke H, Ehwald R, J. Chromatogaphy 708, 263-271) festgestellt wurde (siehe Fig. 4). Die so hergestellte asymmetrische Kompositmembran besitzt eine hohe Permeabilität für Glucose (1-3 µm). Auf einen Objektträger aus Glas wurde eine 6 mm breite und 10 mm lange rechteckige auf beiden Seiten klebende perforierte Folie ("transfer film ARcare 91005" mit einer Dicke von 50 µM, bezogen von Adhesive Research Ireland Ltd.) geklebt. Die Folie enthielt 6 kreisförmig ausgestanzte Löcher mit einem Durchmesser von 2 mm und war 50 µm stark. In jedes der Löcher wurde ein 500 nl - Tropfen einer Lösung von FITC-markiertem Rinderserumalbumin (0.1 %) und Dextran mit einer Molmasse von 2000 kDa (0,7 %) in 0,9 % iger NaCl-Lösung dosiert. Nach dem Trocknen war mit Hilfe des Fluoreszenzmikroskops festzustellen, dass sich das fluoreszierende Protein am ringförmigen Trocknungsrand des Mikrotropfens konzentriert hatte. Die asymmetrische Kompositmembran wurde an die perforierte Klebfolie mit der gelfreien Seite angepresst. Die Membranfläche außerhalb der Klebfläche wurde mit einem Skalpell entfernt. Der Objektträger mit den so entstandenen Dialyseräumen wurde in eine zuvor entgaste Phosphatpufferlösung eingestellt. Nach kurzer Zeit hatten sich auf Grund der starken osmotischen Wirksamkeit der Dextranmoleküle die fluoreszierenden Polymere aufgelöst und im Raum des Mikrodialysekammersystems verteilt. Die fluoreszierenden Proteinmoleküle erwiesen sich als dauerhaft immobilisiert. Die gequollene Kompositmembran ist optisch transparent; Fluoreszenzmikroskopie ist von beiden Seiten des Mikrodialysekammersystems möglich. Damit werden die wesentlichen Vorteile der Erfindung - praktikable Integrierbarkeit eines Mikrodialysekammersystems mit eingeschlossenen kolloidalen Stoffen in Mikrostrukturen an Feststoffoberflächen, effiziente und verlustfreie Immobilisierung von Kolloiden im löslichen und austauschfähigen Zustand bei definierter Schichttdicke im Mikrometerbereich, reproduzierbares, praktikables Herstellungsverfahren, schnelle Auflösung und Verteilung der kolloidalen Sensormoleküle nach dem Einbringen in Wasser - eindeutig bewiesen.

### Beispiel 2

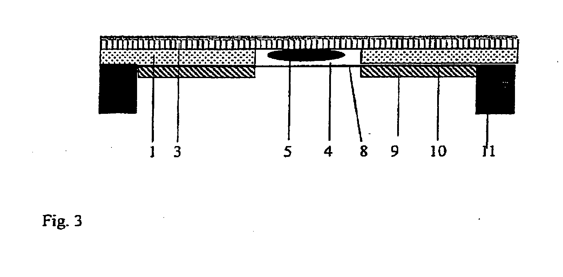

### Herstellung eines Membranosmometers mit miniaturisierter Messzelle - Fig. 3

Die asymmetrische Kompositmembran 3 deckt eine Perforation auf einem 0,2 mm starken plattenartigen Festkörper 1 aus Glas oder Keramik ab, während die andere Seite der Perforation durch die Biegeplatte 8 eines Drucksensors abgedeckt wird. Letztere ist an den perforierten plattenartigen Festkörper 1 geklebt. Der durch die Perforation geschaffene Raum dient als Dialysekammer 4. In ihr befindet sich ein gut wasserlösliches, entwässertes Kolloid 5, z.B. Dextran mit einer Molmasse von 200 kDa. Die eingeschlossene Masse des Hydrokolloids beträgt im Fall des genannten Dextrans vorzugsweise 5 bis 25 % der Menge bzw. Masse von Wasser, die zur vollständigen Füllung des Dialyseraums erforderlich ist. Die gegen die Dialysekammer elektrisch isolierte Biegeplatte 8 ist nach außen an einen plattenförmigen Körper 9 aus einem festen, isolierenden Stoff, z.B. Keramik, geklebt und mit elektrischen Leitern 10 und elektrischen Anschlüssen 11 verbunden. Letztere sind zur Messung des deformationsabhängigen elektrischen Widerstandes der Biegeplatte erforderlich. Als asymmetrische Kompositmembran 3 wird die in Beispiel 1 beschriebene Kompositmembran eingesetzt, deren gerüstbildende Schicht aus kristallinem Aluminiumoxid besteht und das Xerogel ein Polyelektrolytkomplex ist, der die Poren der Membran an der dem Dialyseraum abgewandten Seite bis zu einer Tiefe von ca. 3 µm erfüllt.
Die asymmetrische Kompositmembran wird zunächst mit entgastem Wasser in Verbindung gebracht. Dies führt dazu, dass unter Auflösung des in der Dialysekammer befindlichen Gases Wasser in die Dialysekammer eindringt und die wasserlöslichen Kolloide auflöst. Hierdurch entsteht eine kolloidosmotische Druckdifferenz mit einem Überdruck gegenüber Atmosphärendruck in der Dialysekammer. Bei konstanter Temperatur und konstanter Eintauchtiefe wird ein konstanter, von der Konzentration des Kolloids in der Dialysekammer abhängiger Messwert am Drucksensor gemessen. Tauscht man das Wasser gegen die Lösung eines kolloidalen Stoffes, für den die Membran undurchlässig ist, aus, reduziert sich dieser Wert in Abhängigkeit von der Konzentration des kolloidalen Stoffes in der Lösung. Die Druckerniedrigung im Vergleich zur Messung im Wasser entspricht dem kolloidosmotischen Druck der Lösung.

Der Vorteil der geschilderten Anordnung besteht darin, dass die asymmetrische Kompositmembran auf Grund des kristallinen Aufbaus der gerüstbildenden Schicht eine hohe Biegungsfestigkeit und wegen der Kürze der Diffusionsstrecke durch das gequollene Xerogel (ca. 3 µm) eine hohe Permeabilität für Wasser und niedermolekulare Stoffe besitzt. Hierdurch und durch die geringe Diffusionsstrecke bis zum Grund der Mikrodialysekammer ergibt sich eine schnelle Einstellung des Gleichgewichtsdruckes bzw. des kolloidosmotischen Druckes der äußeren Lösung. Die eingeschlossenen hydrophilen Kolloide beschleunigen die Füllung der Dialysekammer mit Flüssigkeit und sichern eine Überdrucksituation im Membranosmometer, durch die der Messbereich stark erweitert wird.

## Patentansprüche

1. Asymmetrische Kompositmembran für die Mikrodialyse, umfassend eine gerüstbildende poröse Schicht (7) mit einem die Poren einseitig ausfüllenden, in Wasser quellfähigen organischen Xerogel (6), **dadurch gekennzeichnet, dass**
- die gerüstbildende poröse Schicht (7) aus einem rigiden, in Wasser nicht quellfähigen Stoff besteht und 20 bis 120 µm dick ist,
- das Xerogel (6) die Poren nur bis zu einer Tiefe von 10 µm ausfüllt,
- die Poren durchgehend und unverzweigt sowie 20 bis 1000 nm weit sind,
- der Anteil der Porenfläche an der Hauptfläche der porösen Schicht über 25 % beträgt, und
- das Xerogel (6) ein Polyelektrolytkomplex aus Zellulosesulfat und Dimethyldiallylammoniumchlorid oder Chitosan ist, der im gequollenen Zustand für Proteine mit Molmassen über 25 kDa nicht permeabel ist.

2. Mikrodialysekammersystem umfassend
- einen Grundkörper (1) aus einem in Wasser nicht quellfähigen festen Stoff,
- mindestens einen Hohlraum, der als Dialyseraum (4) fungiert und der wasserlösliche kolloidale Stoffe (5) enthält, sowie mindestens eine als Dialysemembran fungierende asymmetrische Kompositmembran (3) gemäß Anspruch 1, wobei
- die asymmetrische Kompositmembran den mindestens einen Dialyseraum (4) abdeckt und
- die Poren der Schicht (7) in ihrem Mündungsbereich auf der dem Dialyseraum (4) abgekehrten Seite das Xerogel (6) enthalten.

3. Mikrodialysekammersystem nach Anspruch 2, **dadurch gekennzeichnet, dass** der mindestens eine Dialyseraum (4), der die wasserlöslichen kolloidalen Stoffe (5) enthält, durch mindestens eine Kavität in dem Grundkörper (1) oder durch Perforation des Grundkörpers oder in einer ihn bedeckenden perforierten Folie (2) oder den Hohlraum der Poren der asymmetrischen Kompositmembran, die sich nahe am Grundkörper (1) befinden, gebildet wird.

4. Mikrodialysekammersystem nach einem der Ansprüche 2 oder 3, **dadurch gekennzeichnet, dass** die kolloidalen Stoffe (5) Affinitätsrezeptoren oder Enzyme umfassen.

5. Mikrodialysekammersystem nach einem der Ansprüche 2 bis 4, **dadurch gekennzeichnet, dass** der feste Grundkörper (1) aus Kunststoff, Glas, Silizium oder Metall besteht.

6. Mikrodialysekammersystem nach einem der Ansprüche 2 bis 5, **dadurch gekennzeichnet, dass** die poröse Schicht (7) aus einem keramischen Material, Metall, Metalloxid, Si, SiO₂, oder einem Kunststoff besteht.

7. Mikrodialysekammersystem nach einem der Ansprüche 2 bis 6, **dadurch gekennzeichnet, dass** die Distanz zwischen der äußeren Oberfläche der Kompositmembran (3) und dem Grund des Dialyseraumes (4) ≤ 0,6 mm ist.

8. Mikrodialysekammersystem nach einem der Ansprüche 2 bis 7, **dadurch gekennzeichnet, dass** das Xerogel (6) die Poren bis zu einer Tiefe von 5 µm. ausfüllt.

9. Mikrodialysekammersystem nach einem der Ansprüche 2 bis 8, **dadurch gekennzeichnet, dass** die Poren 50 bis 250 nm weit sind.

10. Verfahren zur Herstellung eines Mikrodialysekammersystems gemäß einem der Ansprüche 2 bis 9, **dadurch gekennzeichnet, dass**
- kolloidale Stoffe (5) in mindestens einen Dialyseraum (4) oder auf die vom Xerogel (6) freie Oberfläche der Kompositmembran (3) in der zum Abdecken des Dialyseraums (4) vorgesehenen Position in fester oder flüssiger Form dosiert werden,
- die Flüssigkeit verdunstet wird und
- die asymmetrische Kompositmembran (3) über den Dialyseraum (4) fixiert wird.

11. Verwendung eines Mikrodialysekammersystems gemäß einem der Ansprüche 2 bis 9 in der Analytik.

## Claims

1. Asymmetric composite membrane for microdialysis comprising a frame building porous bed (7) with an organic xerogel (6) onesided filled within the pores that is swelling in water (6), in which
- the frame bilding porous bed (7) consists of a rigid material that is not swellable in water and has a thickness between 20 and 120 µm.
- the xerogel (6) infills the pores up to a depth of 10 µm only,
- the pores having a width of 20 to 1000 nm are contiuous and not branched,
the percentage of the pore area in the the whole area of the porous bed is larger than 25 %,
- the xerogel (6) is a polyelectrolyte complex built from cellulose sulphate and dimethylallylammonium chloride or chitosan, which in the swollen state is impermeable to proteins with a molar mass above 25 kDa.

2. Microdyalis chamber system comprising
- a basic chassis (1) made from a solid material not swelling in water,
- at least on cavity representing the dialysis compartment (4) containing water soluble colloidal materials (5),
- as well as at least one asymmetric composite membrane according to claim 1 representing the dialysis membrane (3) according to claim 1 in which
- the asymmetric composite membrane covers the at least one dialysis compartment (4),
- and
- the pores of the bed (7) contain the xerogel (6) in their area of opening at that face which is opposite to the dialysis compartment.

3. Microdyalis chamber system according to clame 2 wherein the at least one dialysis space (4) containing the watersoluble colloidal materials (5) is built by at least one cavity in the basic chassis (1) or by perforation of the basic chassis or a perforated foil covering the basic chassis (2) or the or the empty space formed by the pores of the asymmetric composite membrane being arragned close to basic chassis (1).

4. Microdialysis chamber system according to claim 1 or claim 2, wherein the colloidal materials (5) are affinity receptors or enzymes.

5. Microdialysis chamber system according to one of the claims 1 to 3, wherein the solid chassis (1) consists of a synthetical, glas, silica or metal.

6. Microdialysis chamber system according to one of the claims 1 to 4, wherein the porous bed (7) consists of a ceramic material, metal, metal oxide, Si, SiO₂, or a synthetical.

7. Microdialysis chamber system according to one of the claims 1 bis 5, wherein the distance between the outer surface of the composite membrane (3) and the ground of the dialysis compartment (4) is below 0,6 mm..

8. Microdialysis chamber system according to one of the claims 1 to 6, wherein the xerogel (6) infills the pores up to a depth of 5 µm.

9. Microdialysis chamber system according to one of the claims 1 to 7, wherein the pores in have a width between 50 and 250 nm.

10. Process for building a microdialysis chamber system according to one of the claims 2 to 9, wherein
- colloidal materials (5) are charged in solid or liquid state into a at least one dialysis compartment (4) or on that surface of the composite membrane which is free of the organic xerogel (6) in the position provided to be exposed to the dialysis compartment,
- the liquid is evaporated, and
- the asymmetric dialysis membrane is fixed above the dialysis compartment

11. Use of a microdialysis chamber system according to one or the claims 2 to 9 for analytical purposes.

## Revendications

1. Membrane composite asymétrique pour la microdialyse, comprenant une couche poreuse (7) formant squelette avec un xérogel organique (6) remplissant les pores d'un côté, pouvant gonfler dans l'eau, **caractérisée en ce que**
- la couche poreuse (7) formant squelette se compose d'une matière ne pouvant pas gonfler dans l'eau et est d'une épaisseur de 20 à 120 µm,
- le xérogel (6) remplissant les pores seulement jusqu'à une profondeur de 10 µm,
- les pores étant d'un seul tenant et sans ramifications ainsi que d'une largeur de 20 à 1000 nm,
- le pourcentage en surface de pores à la surface principale de la couche poreuse étant de plus de 25%, et
- le xérogel (6) étant un complexe de polyélectrolytes en sulfate cellulosique et chlorure de diméthyldiallylammonium ou chitosan, lequel est imperméable pour les protéines avec des masses molaires de plus de 25 kDa à l'état gonflé.

2. Système de chambre de microdialyse comprenant
- un corps de base (1) en une matière solide ne pouvant pas gonfler dans l'eau,
- au moins un espace creux, lequel fait office de compartiment de dialyse (4) et contient des matières colloïdales (5) solubles dans l'eau, ainsi qu'au moins une membrane composite asymétrique (3) selon à revendication 1, faisant office de membrane de dialyse, moyennant quoi
- la membrane composite asymétrique recouvre l'au moins un compartiment de dialyse (4), et
- les pores de la couche (7) contenant le xérogel (6) dans leur zone d'embouchure du côté tournant le dos au compartiment de dialyse (4).

3. Système de chambre de microdialyse selon la revendication 2, **caractérisé en ce que** l'au moins un compartiment de dialyse (4), lequel contient les matières colloïdales (5) solubles dans l'eau, est formé par au moins une cavité dans le corps de base (1) ou par perforation du corps de base ou dans un film perforé (2) le recouvrant ou par l'espace creux des pores de la membrane composite asymétrique, laquelle se trouve à proximité du corps de base (1).

4. Système de chambre de microdialyse selon l'une des revendications 2 ou 3, **caractérisé en ce que** les matières colloïdales (5) comprennent des récepteurs d'affinité ou des enzymes.

5. Système de chambre de microdialyse selon l'une des revendications 2 à 4, **caractérisé en ce que** le corps de base solide (1) se compose de plastique, verre, silicium ou de métal.

6. Système de chambre de microdialyse selon l'une des revendications 2 à 5, **caractérisé en ce que** la couche poreuse (7) se compose d'un matériau céramique, de métal, d'oxyde métallique, de Si, SiO₂, ou d'une matière plastique.

7. Système de chambre de microdialyse selon l'une des revendications 2 à 6, **caractérisé en ce que** la distance entre la surface extérieure de la membrane composite (3) et le fond du compartiment de dialyse (4) est ≤ 0,6 mm.

8. Système de chambre de microdialyse selon l'une des revendications 2 à 7, **caractérisé en ce que** le xérogel (6) remplit les pores jusqu'à une profondeur de 5 µm.

9. Système de chambre de microdialyse selon l'une des revendications 2 à 8, **caractérisé en ce que** les pores sont d'une largeur de 50 à 250 nm.

10. Procédé pour la fabrication d'un système de chambre de microdialyse selon l'une des revendications 2 à 9, **caractérisé en ce que**
- des matières colloïdales (5) sont dosées sous forme solide ou liquide dans au moins un compartiment de dialyse (4) ou bien sur la surface libre de xérogel (6) de la membrane composite (3) dans la position prévue pour recouvrir le compartiment de dialyse (4),
- **en ce que** l'on fait s'évaporer le liquide et
- **en ce que** l'on fixe la membrane composite asymétrique (3) au-dessus du compartiment de dialyse (4).

11. Utilisation d'un système de chambre de microdialyse selon l'une quelconque des revendications 2 à 9 en analytique.
